# EUROPÄISCHE PATENTSCHRIFT

(11) **EP 2 603 074 B1**
(45) Veröffentlichungstag und Bekanntmachung des Hinweises auf die Patenterteilung: **24.02.2016**
(21) Anmeldenummer: 11748265.3
(22) Anmeldetag: 30.07.2011
(51) Int. Cl.: A01N 1/02, C12N 5/071

(54) **VERFAHREN ZUR HERSTELLUNG DESINFIZIERTER HUMANER ZELLSUSPENSIONEN**
PROCESS FOR THE PREPARATION OF DESINFECTED HUMAN CELL SUSPENSIONS
PROCEDEE POUR LA PREPARATION DES SUSPENSIONS DE CELLULES DESINFECTEES

(30) Priorität: 14.08.2010 DE 102010034330
(43) Veröffentlichungstag der Anmeldung: 19.06.2013
(73) Patentinhaber: Cytonet GmbH & Co. KG, 69469 Weinheim (DE)
(72) Erfinder: ALEKSANDROVA, Krasimira, 30625 Hannover (DE); BARTHOLD, Marc, 30171 Hannover (DE); ARSENIEV, Lubomir, 30625 Hannover (DE); GRIESEL, Carsten, 68526 Ladenburg (DE); PRIESNER, Christoph, 30655 Hannover (DE)
(74) Vertreter: Schrell, Andreas
(86) Internationale Anmeldenummer: PCT/EP2011/003838
(87) Internationale Veröffentlichungsnummer: WO 2012/022429

(56) Entgegenhaltungen:
- WO-A1-92/12632
- US-A1- 2003 021 775
- US-A1- 2004 110 289
- US-A1- 2010 047 213
- GERLACH JOERG C ET AL: "Comparison of four methods for mass hepatocyte isolation from pig and human livers", TRANSPLANTATION, WILLIAMS AND WILKINS, BALTIMORE US, Bd. 57, Nr. 9, 1. Januar 1994 (1994-01-01) , Seiten 1318-1322, XP009154462, ISSN: 0041-1337
- ALEXANDROVA KRASSIMIRA ET AL: "Large-scale isolation of human hepatocytes for therapeutic application", CELL TRANSPLANTATION, US, Bd. 14, Nr. 10, 1. Januar 2005 (2005-01-01), Seiten 845-853, XP009154317, ISSN: 0963-6897
- MITRY RAGAI R ET AL: "Human hepatocyte isolation and relationship of cell viability to early graft function", CELL TRANSPLANTATION, US, Bd. 12, Nr. 1, 1. Januar 2003 (2003-01-01) , Seiten 69-74, XP009154318, ISSN: 0963-6897
- E. FITZPATRICK ET AL: "Human hepatocyte transplantation: state of the art", JOURNAL OF INTERNAL MEDICINE, Bd. 266, Nr. 4, 1. Oktober 2009 (2009-10-01), Seiten 339-357, XP55012817, ISSN: 0954-6820, DOI: 10.1111/j.1365-2796.2009.02152.x
- UMBERTO BACCARANI ET AL: "Isolation of human hepatocytes from livers rejected for liver transplantation on a national basis: Results of a 2-year experience", LIVER TRANSPLANTATION, SAUNDERS, PHILADELPHIA, PA, US, Bd. 9, Nr. 5, 1. Mai 2003 (2003-05-01), Seiten 506-512, XP008093068, ISSN: 1527-6465, DOI: 10.1053/JLTS.2003.50087
- M. N. BERRY: "HIGH-YIELD PREPARATION OF IHIGH-YIELD PREPARATION OF ISOLATED RAT LIVER PARENCHYMAL CELLS: A Biochemical and Fine Structural Study", THE JOURNAL OF CELL BIOLOGY, Bd. 43, Nr. 3, 1. Dezember 1969 (1969-12-01), Seiten 506-520, XP55013428, ISSN: 0021-9525, DOI: 10.1083/jcb.43.3.506 in der Anmeldung erwähnt

## Beschreibung

Die vorliegende Erfindung betrifft Verfahren zur Herstellung von desinfizierten Einzelzellpräparaten und die derart hergestellten Präparate.

Lebertransplantationen sind die wichtigste lebensrettende Maßnahme bei akutem oder chronischem Leberversagen. Um dem allein durch Spenderorgane nicht zu deckenden Bedarf an Transplantationsmaterial zu begegnen, wurden in den letzten Jahren alternative Methoden zur kompletten Organtransplantation entwickelt, zum Beispiel die Teillebertransplantation oder die Transplantation von Leberzellpräparaten.

Besonders attraktiv sind Einzelzellpräparate deshalb, weil sie aus für die Organtransplantation ungeeigneten Spenderorganen gewonnen werden können. Zur Gewinnung von Hepatozyten aus Lebergewebe ist eine Zwei-Schritt-Perfusionsmethode bekannt, die einen Kollagenase-haltigen und einen EDTA-haltigen Puffer einsetzt (Berry und Friend, 1969, The Journal of Cell Biology, Vol. 43, Seiten 506-520). Hierbei werden durch die Perfusion des Lebergewebes mit dem Kollagenase-haltigen Puffer einzelne Zellen enzymatisch aus dem Gewebeverband entlassen.

Da die gewonnenen Einzelzellpräparate Arzneimittel darstellen, muss die mikrobielle Sterilität in jedem Fall gewährleistet sein. Dies ist jedoch in cirka 70 bis 80 % der Präparate nicht der Fall, da eine Kontamination meist bereits im Transportmedium des Organs vorliegt, die in der Regel aus dem Organ- beziehungsweise Gewebematerial des Spenders stammt. Bei einem so wertvollen Gut wie humanen Spenderorganen ist es jedoch wünschenswert, eine große Ausbeute an mikrobiell nicht belasteten, transplantierbaren Gewebe- oder Zellpräparaten zu erhalten. Herkömmliche Dekontaminationsverfahren wie Erhitzen, Autoklavieren oder Bestrahlen stehen einem Erhalt der Lebensfähigkeit der Zellen entgegen und können daher für die Desinfektion des Gewebes nicht verwendet werden.

Verfahren zur Dekontaminiation von Gewebe mittels Antibiotika sind ebenfalls bereits bekannt. Sie wurden zum Beispiel für die Lagerung von Cornea-Transplantaten (US 4,695,536) oder die Dekontamination von Herzklappentransplantaten entwickelt (WO 92/12632 und EP 0 889 690). Die Einwirkzeiten der hierbei verwendeten antibiotischen Zusammensetzungen reichen von 24 Stunden bis hin zur Lagerung über mehrere Wochen. Selbst eine Einwirkzeit von 24 Stunden ist jedoch für hochsensible Gewebe wie Leber ungeeignet. Außerdem erfolgt die Dekontamination bei diesen Verfahren nur oberflächlich. Eine weitere Anforderung an Antibiotika-basierte Dekontaminationsverfahren für Gewebe und Zellen liegt darin, dass das Verfahren sowie die darin eingesetzten Mittel weder die Gewinnung der Leberzellen noch deren Zellqualität und Lebensfähigkeit negativ beeinflussen dürfen und mit dem zur Qualitätskontrolle eingesetzten Sterilitätsnachweissystem kompatibel sein müssen.

Das der vorliegenden Erfindung zugrundeliegende technische Problem liegt also darin, Verfahren zur schonenden und wirksamen Dekontamination, das heißt Desinfektion, von Geweben bereitzustellen, die die vorstehend erwähnten Nachteile überwinden, insbesondere solche, die eine wirksamere und schnellere Desinfektion des Gewebes unter besonders schonenden Bedingungen, insbesondere die gleichzeitige Aufrechterhaltung der Lebensfähigkeit der Gewebe oder Zellen gewährleisten, selbst, wenn diese Gewebe oder darin enthaltene Zellen sehr empfindlich sind, also zum Beispiel aus der Leber stammen.

Das der vorliegenden Erfindung zugrundeliegende technische Problem wird gelöst durch die Lehre gemäß der unabhängigen Patentansprüche. Die vorliegende Erfindung betrifft demgemäß ein Verfahren zur Herstellung von desinfizierten Zellpräparaten aus Geweben von Säugern, umfassend die folgenden Verfahrensschritte: a) Perfusion von Gewebe aus Säugern mit einer enzymfreien flüssigen in mindestens einem Perfusionspuffer vorliegenden, mindestens ein Antibiotikum aufweisenden antibiotischen Zusammensetzung, b) Durchführung einer enzymatischen antibiotikafreien Behandlung des Gewebes zum Erhalt einer Einzelzellsuspension und c) Erhalt von desinfizierten Zellpräparaten, wobei die enzymatische Behandlung gemäß Schritt b) im Anschluss an Schritt a) erfolgt.

Die vorliegende Erfindung basiert auf einem Verfahren zur Herstellung von desinfizierten Präparaten, insbesondere Einzelzellpräparaten, aus Geweben von Säugern, insbesondere Menschen, umfassend die folgenden Verfahrensschritte: a) die Perfusion von Gewebe aus Säugern mit einer flüssigen antibiotischen Zusammensetzung, b) die Durchführung einer enzymatischen Behandlung des Gewebes, insbesondere zum Erhalt einer Einzelzellsuspension, und c) den Erhalt von desinfizierten Präparaten, insbesondere Einzelzellpräparaten, also Präparaten, in denen die Zellen nicht mehr in einem Gewebeverbund, sondern isoliert voneinander als einzelne Zellen oder ggf. als Zellcluster oder kleinere Zellaggregate vorliegen. Die Erfindung stellt demgemäß in vorteilhafter Weise ein Verfahren zur Verfügung, gemäß dem aus Geweben, also z. B. Zellverbänden oder Organen, von Säugern, insbesondere Menschen, in einem in vitro-Verfahren desinfizierte Präparate, insbesondere Einzelzellpräparate, vorzugsweise Einzelzellsuspensionen, hergestellt werden können. Vorteilhafterweise kann erfindungsgemäß ein Zellpräparat erhalten werden, das sich besonders gut für eine aussagekräftige Steriltestung eignet. Die erfindungsgemäße Verfahrensweise erlaubt daher eine Steriltestung, die eine hohe Aussagekraft zur Sterilität der erhaltenen Präparate bereitstellt.

Die Erfindung geht von bereitgestellten Geweben aus Säugern aus, die beispielsweise aus lebenden oder toten Säugern, insbesondere erst kürzlich gestorbenen Säugern, stammen können. In besonders bevorzugter Ausführungsform werden unter Säugern Menschen oder Tiere, z. B. Nutztiere oder Versuchstiere, insbesondere Schweine, Kühe, Pferde, Hunde, Katzen, Schafe, Ziegen, Affen, Primaten, Vögel oder Nagetiere verstanden.

Die aus den Spendersäugern entnommenen und derart für die vorliegende Erfindung bereitgestellten Gewebe, insbesondere Zellverbände oder Organe, werden als Ausgangsmaterial in der vorliegenden Erfindung eingesetzt. Die Erfindung sieht vor, dass derartig bereitgestellte Gewebe mit einer antibiotischen Zusammensetzung in Kontakt gebracht und perfundiert werden, vorzugsweise unter Nutzung des Gewebe-, insbesondere Organ-eigenen Gefäßsystems. Die Erfindung sieht ferner vor, dass anschließend an die Perfusion mit der antibiotischen Zusammensetzung das Gewebe einer enzymatischen Behandlung unterzogen wird, im Verlauf derer der Gewebeverband aufgelöst und eine Einzelzellsuspension erhalten wird. Auf diese Art und Weise wird erreicht, dass Einzelzellen vorliegen, die demgemäß besonders effektiv antibiotisch behandelt und dadurch desinfiziert werden können.

Im Zusammenhang mit der vorliegenden Erfindung wird unter einer Einzelzellpräparation oder einer Einzelzellsuspension insbesondere eine Präparation oder Suspension verstanden, in der die Zellen einzeln und ohne permanenten physischen Kontakt zueinander vorliegen. In einer weiteren Ausführungsform wird unter einer Einzelzellpräparation oder einer Einzelzellsuspension auch verstanden, dass die überwiegende Zahl der vorhandenen Zellen, insbesondere mehr als 50 %, mehr als 60 %, mehr als 70 %, mehr als 80 %, mehr als 90 % oder mehr als 95 % der Zellen, losgelöst voneinander und ohne permanenten physischen Kontakt zueinander vorliegen und der auf 100 % aufaddierende Rest der Zellen in Form von Zellclustern, kleineren oder größeren Zellaggregaten und nicht vollständig gelösten Resten des Gewebeverbands vorliegt.

Im Zusammenhang mit der vorliegenden Erfindung wird unter einer Perfusion insbesondere eine Gewebeperfusion verstanden, das heißt die Durchströmung eines Hohlorgans beziehungsweise der im Gewebe enthaltenen Gefäße, insbesondere Blutgefäße, mit einer Flüssigkeit, das heißt insbesondere der vorliegenden antibiotischen Zusammensetzung. Im Zusammenhang mit der vorliegenden Erfindung stellt eine Perfusion eine besonders bevorzugte Ausführung einer Inkubation dar.

Im Zusammenhang mit der vorliegenden Erfindung wird unter Gewebe ein zusammenhängender Verband von Zellen, insbesondere gleicher Funktion und/oder Morphologie verstanden, insbesondere auch ein Organ, z. B. eine Leber, ein Organteil, ein Gewebeverband oder Sektionsmaterial, insbesondere aus der Leber, insbesondere Lebersektionsmaterial. Erfindungsgemäß ist das eingesetzte Gewebe Lebergewebe, z. B. eine vollständige Leber oder ein Teil davon, z. B. Lebersektionsmaterial. Die erfindungsgemäß hergestellten Einzelzellsuspensionen sind bevorzugt Leberzellsuspensionen.

Erfindungsgemäß wird zunächst die Perfusion mit einer enzymfreien antibiotischen, vorzugsweise in mindestens einem Perfusionspuffer vorliegenden, Zusammensetzung durchgeführt und anschließend eine enzymatische antibiotikafreie Behandlung gemäß Schritt b) vorgenommen im Rahmen derer der Gewebeverband aufgelöst und Einzelzellen des in Schritt a) eingesetzten Gewebes erhalten werden, so dass im Verfahrensschritt c) vollständig desinfizierte Einzelzellen erhalten werden.

Erfindungsgemäß kann auch in einem optionalen Verfahrensschritt vorgesehen sein, das eingesetzte Gewebe vor der erfindungsgemäßen Perfusion oberflächlich mit einer antibiotischen Zusammensetzung, vorzugsweise der erfindungsgemäß eingesetzten antibiotischen Zusammensetzung zu spülen, insbesondere ohne zu perfundieren, also eine primäre oberflächliche Dekontamination durchzuführen. Hierbei beträgt die Inkubationszeit vorzugsweise nur 5 bis 60 Minuten, bevorzugt 5 bis 45 Minuten, besonders bevorzugt 5 bis 30 Minuten. Gegebenenfalls kann bei einer so kurzen Inkubationszeit auch eine höhere Antibiotika-Konzentration eingesetzt werden als während der Perfusion. Die oberflächliche Spülung kann auch während der Verfahrensschritte a) und/oder b) durchgeführt werden.

Die erfindungsgemäß vorgesehene enzymatische Behandlung des Gewebes zum Erhalt einer Einzelzellsuspension setzt vorzugsweise mindestens ein oder vorzugsweise mindestens zwei Enzyme ein, die sich in einer vorzugsweise wässrigen Lösung, insbesondere gepufferten Lösung, befinden. Die für die enzymatische Behandlung eingesetzten Enzyme sind in der Lage, den Gewebeverband aufzulösen, insbesondere Zell-Zell-Kontakte und Zell-extrazellulare Matrix-Kontakte zu lösen, und aus diesem ohne Beschädigung oder Zerstörung der Zellen eine Einzelzellsuspension herzustellen. In erfindungsgemäß bevorzugter Ausführungsform sind besonders geeignete Enzyme Kollagenasen, Proteinasen und Mischungen derselben. Im Zusammenhang mit der vorliegenden Erfindung wird unter einer Proteinase auch eine Protease verstanden.

Bevorzugt sind Mischungen aus Kollagenase und Protease, insbesondere neutrale Proteasen, im Verhältnis enzymatischer Aktivitäten von 1 Einheit Kollagenase zu 40 bis 60, vorzugsweise 50 Einheiten Protease.

Pro Perfusion werden daher bevorzugt ca. 2000 bis 4000 Wünsch-Einheiten Kollagenase eingesetzt in Kombination mit 100 000 Casein-Einheiten oder mit 400 bis 2500 Clostridiopeptidase I Einheiten.

In einer besonders bevorzugten Ausführungsform des erfindungsgemäßen Verfahrens wird das Gewebe also in vitro mit der Zusammensetzung, die mindestens ein Enzym zur Auflösung des Gewebes in Einzelzellen enthält, insbesondere a) mindestens eine Kollagenase oder b) mindestens eine Kollagenase und mindestens eine Proteinase, insbesondere neutrale Proteinase, enthält, perfundiert. Dies führt zur Loslösung einzelner Zellen aus dem Gewebeverband und damit zum Erhalt von Einzelzellpräparaten. Das heißt, die Kollagenase oder die Kollagenase und Proteinase liegen getrennt von dem mindestens einen antibiotischen Wirkstoff, bevorzugt den antibiotischen Wirkstoffen, in dem mindestens einen Perfusionspuffer, bevorzugt Phosphat-HEPES-Puffer, in einem eigenen Lösemittel, zum Beispiel einem Puffer, vor und werden getrennt vom antibiotischen Perfusionsschritt, das heißt anschließend, eingesetzt.

Gemäß der Erfindung liegt die erfindungsgemäß eingesetzte antibiotische Zusammensetzung in flüssiger Form vor, zum Beispiel als Lösung oder Suspension, vorzugsweise als wässrige Lösung oder Suspension, besonders bevorzugt in mindestens einem Perfusionspuffer.

In besonders bevorzugter Ausführungsform weist die flüssige antibiotische Zusammensetzung mindestens ein, bevorzugt mindestens zwei, insbesondere mindestens drei, vorzugsweise mindestens vier oder auch mehr unterschiedliche Antibiotika auf. Diese können ausgewählt sein aus der Gruppe bestehend aus den Nicht-Penicillinen der ß-Lactam-Antibiotika, Aminoglykosiden, Glykopeptid-Antibiotika und Polypeptid-Antibiotika.

Die erfindungsgemäß eingesetzte antibiotische Zusammensetzung weist in bevorzugter Ausführungsform keinen negativen oder inhibitorischen Effekt auf die eingesetzte enzymatische Behandlung, insbesondere Kollagenase- oder Proteinase-Behandlung auf.

Es ist gemäß der vorliegenden Erfindung vorgesehen, dass die antibiotische Zusammensetzung frei von Kollagenasen und Proteinasen ist.

Die erfindungsgemäß bevorzugt eingesetzte antibiotische Zusammensetzung, insbesondere Antibiotikakombination, zeichnet sich vorteilhafterweise dadurch aus, dass sie keinen negativen Einfluss auf die Vitalität und Morphologie der isolierten Zellen aufweist.

Das mindestens eine Antibiotikum, insbesondere die Antibiotika, liegen in der erfindungsgemäßen Zusammensetzung in Mengen vor, die bewirken, dass das Wachstum, die Vermehrung und/oder die Lebensfähigkeit von Mikroorganismen wie Bakterien, bevorzugt solchen, die in Transportmedien von Spenderorganen vorkommen, im Wesentlichen reduziert oder gehemmt wird, während die Lebensfähigkeit des Gewebes oder der Zellen im Wesentlichen aufrechterhalten bleibt.

Im Zusammenhang mit der vorliegenden Erfindung wird unter einer antibiotischen Zusammensetzung eine stoffliche Zusammensetzung verstanden, die antibiotische Wirksamkeit entfaltet. Im Zusammenhang mit der vorliegenden Erfindung wird unter einer antibiotischen Wirksamkeit eine Schadwirkung, insbesondere eine antimikrobielle Schadwirkung, wie Abtötung, Fortpflanzungshemmung, Fortpflanzungsreduzierung, Wachstumshemmung oder Wachstumsreduzierung, insbesondere eine abtötende Wirkung auf Mikroorganismen, insbesondere Bakterien, und zwar sowohl gram-positive als auch gram-negative aerobe und anaerobe Bakterien, Pilze, Protozoen oder Fortpflanzungsprodukte derselben, wie Sporen oder Keime, verstanden. Besonders bevorzugt ist die antibiotische Zusammensetzung besonders wirksam gegenüber Mikroorganismen, die in oder auf Säugerspendergewebe oder -organen oder in deren Transportmedien vorkommen.

Im Zusammenhang mit der vorliegenden Erfindung wird unter einer antibiotischen Wirksamkeit daher vorzugsweise verstanden, dass Mikroorganismen abgetötet werden, das heißt die antibiotische Zusammensetzung mikrobizid, vorzugsweise bakteriozid, wirkt. Erfindungsgemäß muss in einer Ausführungsform der Erfindung die antibiotische Wirksamkeit nicht zwangsläufig mit einer mikrobiziden Wirkung einhergehen, sondern kann auch bedeuten, dass die Mikroorganismen lediglich in ihren Lebensfunktionen, zum Beispiel Wachstum, Stoffwechsel oder Fortpflanzung, eingeschränkt werden, das heißt eine mikrobistatische, insbesondere eine bakteriostatische, Wirkung auftritt.

Im Zusammenhang mit der vorliegenden Erfindung wird unter dem Begriff der antibiotischen Wirksamkeit auch bevorzugt eine Desinfektionswirkung verstanden, das heißt in besonders bevorzugter Weise wirkt die antibiotische Zusammensetzung desinfizierend, das heißt befreit die eingesetzten Materialien, zum Beispiel Gewebe und/oder Zellen, von lebenden Mikroorganismen, vorzugsweise vollständig oder nahezu vollständig, beziehungsweise tötet diese ab. Die Desinfektion erfasst dabei nicht nur die Entfernung oder Abtötung von Keimen, das heißt frühen Entwicklungsstadien eines Mikroorganismus, sondern auch von allen anderen Entwicklungs- und Zustandsstadien von Mikroorganismen inklusive deren Dauerformen oder infektiösen Teilen der Mikroorganismen. Unter einer Desinfektion wird auch eine signifikante Reduktion der Anzahl an vermehrungsfähigen Mikroorganismen verstanden, insbesondere, dass der Restgehalt an vermehrungsfähigen Mikroorganismen in einer Einheit des Desinfekionsgutes höchstens 10⁻⁶ Kolonie-bildende Einheiten beträgt. Im Zusammenhang mit der vorliegenden Erfindung wird unter einer Desinfektion auch eine Dekontamination verstanden.

In besonders bevorzugter Ausführungsform liegt die flüssige antibiotische Zusammensetzung in mindestens einem Perfusionspuffer vor.

Die vorliegende Erfindung betrifft also ein Verfahren, in dem eine antibiotische Zusammensetzung eingesetzt wird, umfassend, vorzugsweise bestehend aus, Nicht-Penicillinen der ß-Lactam-Antibiotika, Aminoglykosiden, Glykopeptid-Antibiotika und/oder Polypeptid-Antibiotika in vorzugsweise mindestens einem Perfusionspuffer. Die erfindungsgemäß eingesetzte antibiotische Zusammensetzung ist geeignet, insbesondere geeignet und bestimmt, zur in vitro-Desinfektion von Säugergewebe und Säugerzellen, bevorzugt menschlichem Gewebe, Organen oder Organteilen und menschlichen Zellen, insbesondere Lebergewebe und Leberzellen. Die Desinfektion des isolierten Gewebes wird durch Perfusion mit der erfindungsgemäß eingesetzten antibiotischen Zusammensetzung erreicht, wobei das natürliche Gefäßsystem genutzt wird. Hierdurch wird eine mikrobielle Desinfektion des gesamten, auch inneren und schwer zugänglichen Gewebes, und nicht nur dessen Oberfläche erreicht. Dies ist vor allem bei der Leber besonders wichtig, da beim sterbenden Patienten in unterschiedlichem Ausmaß Bakterien die Darmwände durchwandern und so eventuell über das Pfortadersystem des zu entnehmenden Organs durch die Perfusion eingeschwemmt werden. Ebenso kann es zu einer retrograden Kontamination über das Gallengangsystem kommen, das physiologischerweise in offenem Kontakt mit der Darmflora steht. Trotz optimaler Arbeitstechniken wird eine solche Kontamination daher nie vollständig zu verhindern sein und macht eine schonende und wirksame Dekontamination mit der erfindungsgemäß eingesetzten antibiotischen Zusammensetzung besonders notwendig. Darüber hinaus können solche "inneren" Kontaminationen unter Umständen dazu führen, dass die Erreger im Transportmedium zuerst nicht nachweisbar sind, sondern erst im Laufe der enzymatischen Dissoziation des Gewebematerials freigesetzt werden. Eine Desinfektion des isolierten Gewebes durch Perfusion mit der erfindungsgemäßen antibiotischen Zusammensetzung führt daher vorteilhafterweise zu einer frühzeitigen Eliminierung solcher "inneren" Kontaminationen. Ohne Zugabe von Antibiotika sind somit nahezu 100% der Leberzellpräparate, hergestellt aus Leberorganen mit kontaminiertem Transportmedium, ebenfalls kontaminiert. Durch die erfindungsgemäße Dekontamination konnten jedoch über 65 % desinfizierte Leberzellpräparate hergestellt werden.

Vorzugsweise wird das Gewebe in mindestens einem Perfusionsschritt, vorzugsweise zwei, drei, vier oder mehreren Perfusionsschritten, zum Beispiel drei Schritten perfundiert. In bevorzugter Ausführungsform können pro Perfusionsschritt jeweils 3 bis 5 Liter Antibiotika-haltiger oder Kollagenase/Proteinase-haltiger Perfusionspuffer, bevorzugt Phosphat-HEPES-Puffer, eingesetzt werden. Erfindungsgemäß bevorzugt ist , dass bei drei Perfusionsschritten nur die ersten beiden Perfusionspuffer antibiotikahaltig sind und die Kollagenase/Proteinase im letzten Perfusionsschritt in einem antibiotikafreien Perfusionspuffer eingesetzt wird.

In einer besonders bevorzugten Ausführungsform liegt die antibiotische Zusammensetzung in mindestens zwei Perfusionspuffern, insbesondere zwei Perfusionspuffern, insbesondere oder nämlich mindestens einem EGTA- oder EDTA-haltigen Phosphat-HEPES-Puffer und mindestens einen Phosphat-HEPES-Puffer oder anderen geeigneten Pufferlösungen vor.

Vorzugsweise sind die für die Perfusion, das heißt Durchströmung, des Gewebematerials verwendeten Perfusionspuffer Phosphat-HEPES (2-(4-(2-Hydroxyethyl)-1 -piperazinyl)-ethansulfonsäure)-Puffer, aber auch EGTA (Ethylenglykol-bis-(aminoethylether)-N, N'-tetraessigsäure)- oder EDTA (Ethylendiamintetraessigsäure)-haltige Phosphat-HEPES-Puffer. Die verwendeten Perfusionspuffer weisen vorzugsweise einen physiologischen pH-Wert von 7 bis 8, besonders bevorzugt 7,3 bis 7,8, insbesondere 7,5 auf.

In besonders bevorzugter Ausführungsform beträgt sowohl die Perfusionszeit als auch die enzymatische Behandlungszeit jeweils 10 bis 360 Minuten, vorzugsweise 20 bis 240 Minuten, insbesondere 30 bis 120 Minuten. In einer weiteren besonders bevorzugten Ausführungsform beträgt die Temperatur sowohl für die Perfusion als auch für die enzymatische Behandlung 9 bis 39°C, vorzugsweise 10 bis 37°C, insbesondere 25 bis 37°C. Während der Perfusion und ggf. der enzymatischen Behandlung wird in einer bevorzugten Ausführungsform die Temperatur der antibiotischen Perfusionszusammensetzung und des perfundierten Organs erhöht, besonders bevorzugt von einer Ausgangstemperatur im Bereich von weniger als 10°C, z. B. 4°C bis 10°C, bis auf eine Endtemperatur im Bereich von 30°C bis 39°C, z. B. auf 37°C.

Durch das Vorhandensein von Kollagenase beziehungsweise Kollagenase und Proteinase wird während der Inkubation, insbesondere Perfusion, eine Dissoziation des Gewebematerials erreicht und es können somit Einzelzellsuspensionen erhalten werden.

Die vorliegende Erfindung stellt in bevorzugter Ausführung auch ein Verfahren zur Desinfektion eines Gewebes oder zur Herstellung eines desinfizierten Zellpräparates aus Säugern in vitro bereit, wobei a) das Gewebe aus Säugern mit der antibiotischen Zusammensetzung gemäß der vorliegenden Erfindung unter geeigneten Bedingungen, insbesondere für einen geeigneten Zeitraum bei einer geeigneten Temperatur perfundiert, b) anschließend eine enzymatische Behandlung zur Herstellung einer Einzelzellsuspension aus dem Gewebe durchgeführt und c) ein desinfiziertes Zellpräparat erhalten wird.

In einer besonders bevorzugten Ausführungsform der vorliegenden Erfindung ist vorgesehen, die mittels des erfindungsgemäßen Verfahren erhaltenen desinfizierten Zellpräparate anschließend mindestens einem, z. B. einem, zwei, drei oder mehreren, Waschschritt(en), einem Isolierschritt(e) oder beiden zu unterziehen, der bzw. die die Konzentration der eingesetzten Antibiotika, Enzyme oder von beiden reduziert beziehungsweise diese vollständig oder nahezu vollständig aus den erhaltenen Präparaten entfernt. Besonders bevorzugt wird im Anschluss an Verfahrensschritt b), z. B. vor oder nach Verfahrensschritt c) ein oder mehrere Waschschritt(e), vorzugsweise mit einem Antibiotika-freien Waschpuffer durchgeführt. Dies führt vorteilhafterweise dazu, dass die im erfindungsgemäßen Verfahren eingesetzten Antibiotika und Enzyme vollständig oder nahezu vollständig entfernt werden und die erhaltenen Einzelzellpräparationen für eine anschließende Steriltestung geeignet sind.

Es kann in bevorzugter Ausführung vorgesehen sein, dass die gewonnenen desinfizierten Zellpräparate zur Aufbewahrung kryokonserviert werden. Hierzu werden die Präparate in einem zur Kryokonservierung geeigneten Medium bei einer geeigneten tiefen Temperatur gelagert.

Vorteilhafterweise konnte erfindungsgemäß gezeigt werden, dass durch die erfindungsgemäßen Verfahren in erheblich schnellerer Zeit eine höhere Zellausbeute (gewonnene Gesamtzellzahl), eine höhere spezifische Zellausbeute (Zellzahl pro Gramm eingesetztem Lebergewebe), sowie eine höhere Vitalität der isolierten desinfizierten Leberzellen (Anteil der lebenden Zellen in Suspension) erreicht werden konnte.

Die Verwendung der erfindungsgemäß eingesetzten antibiotischen Zusammensetzung führt vorteilhafterweise dazu, dass aus zum Beispiel Spenderorganen, die zur Transplantation zur Verfügung stehen, dafür aber nicht geeignet sind, besonders schnell, schonend und wirksam desinfizierte Einzelzellpräparate, insbesondere bevorzugt Zellsuspensionen in zum Beispiel Kulturmedien gewonnen werden können, die bisher nicht erhältlich waren, insbesondere von Leberzellen.

Die erfindungsgemäße Verfahrensweise weist den Vorteil auf, dass die durchgeführte Antibiotikabehandlung eine anschließend durchgeführte enzymatische Behandlung, insbesondere Kollagenase- und/oder Proteinasebehandlung, nicht beeinträchtigt und darüber hinaus auch anschließend durchgeführte Sterilitätskontrollen nicht negativ beeinflusst. In besonders bevorzugter Ausführungsform eignen sich die erfindungsgemäß eingesetzten antibiotischen Zusammensetzungen daher für die Herstellung von desinfizierten Zellsuspensionen aus Geweben, also nicht im Gewebe- oder Organverband vorliegenden Einzelzellen in Medien. Zum Durchströmen des isolierten Säugergewebes oder des isolierten Organs werden vorzugsweise auf die Größe des Gewebes beziehungsweise Organs angepasste Volumina der erfindungsgemäß eingesetzten in mindestens einem Perfusionspuffer vorliegenden antibiotischen Zusammensetzung eingesetzt. Diese können empirisch ermittelt werden, wie dem Fachmann bekannt ist.

Weitere vorteilhafte Ausgestaltungen der Erfindung ergeben sich aus den Unteransprüchen.

Die Erfindung wird in den nachfolgenden Beispielen näher erläutert. Die Beispiele sind nicht einschränkend zu verstehen, vielmehr sollen die Vorteile der Erfindung näher erläutert und anhand konkreter Beispiele illustriert werden.

### Beispiele

Beispiel 1, nicht erfindungsgemäß: Herstellung desinfizierter Leberzellsuspensionen mit gleichzeitiger Antibiotika- und Enzymbehandlung

### A. Durchführung

Zur Herstellung desinfizierter Leberzellsuspensionen aus einer Leber wurde die folgende antibiotische Zusammensetzung hergestellt:
Die Antibiotika wurden in folgenden Konzentrationen zu allen angegebenen Perfusionspuffern gegeben:

| ANTIBIOTIKUM | KONZENTRATION |
|---|---|
| MEROPENEM | 0,2 mg / ml |
| VANCOMYCIN | 0,05 mg / ml |

Ein für die Ganzorgantransplantation ungeeignetes Multispender-Leberorgan wurde zuerst oberflächlich mit 5 L antibiotikahaltigem Phosphat-HEPES-Puffer für 30 min. bei Raumtemperatur gespült und anschließend in drei Schritten mit folgenden Volumina an antibiotikahaltigen Puffern, pH 7,25 - 7,45, perfundiert:
1. 5 L EDTA-haltigem Phosphat-HEPES-Puffer; Perfusion 10 min; 20 - 40 °C;
2. 5 L Phosphat-HEPES-Puffer; Perfusion 10 min; 25 - 40 °C;
3. 3 L Kollagenase/Protease-haltigem Phosphat-HEPES-Puffer; Perfusion 24 min; 25 - 40 °C

Kollagenase/Protease-Konzentration: Kombination aus 2000 Wünsch-Einheiten Kollagenase I und 400 bis 2500 Einheiten Protease (Clostridiopeptidase I).

Die Perfusionszeit mit den Enzym- und antibiotikahaltigen Puffern betrug insgesamt 44 min. Für die Perfusion wurde das natürliche Blutgefäßsystem der eingesetzten Leber genutzt.

Während der Perfusion wurde die Temperatur des perfundierten Organs und der Umgebung langsam von <10°C auf 37°C erhöht (Puffertemperatur zwischen Raumtemperatur und 40°C).

Die Zellen wurden nach ihrer Isolierung aus dem Gewebe mehrfach mit antibiotikafreiem Puffer gewaschen bis die Antibiotika-Konzentration unter 1/1000 der eingesetzten Antibiotika-Konzentration lag.

Eine Probe zur Sterilkontrolle wurde sowohl von dem Transportmedium, in dem das Organ geliefert wurde, als auch von der desinfizierten Zellsuspension nach der Kryokonservierung entnommen.

### B. Ergebnisse

Im Transportmedium wurden folgende Mikroorganismen nachgewiesen: Enterobacter cloacae, Acinetobacter baumannii, wobei die Zeit der während der automatischen Inkubation, bis mikrobielles Wachstum festgestellt wurde, die so genannte "Time to positivity" 1,67 h betrug.

In der erfindungsgemäß desinfizierten Zellsuspension nach Kryokonservierung konnten auch nach 14-tägiger Bebrütung keine Keime nachgewiesen werden.

Nach einer ausreichenden Anzahl Waschschritte mit antibiotikafreiem Puffer kann mit den hergestellten Zellsuspensionen ein Steriltest durchgeführt werden.

### Beispiel 2: Herstellung desinfizierter Leberzellsuspensionen mit sequenzieller Antibiotika- und Enzymbehandlung

### A. Durchführung

Ein für die Ganzorgantransplantation ungeeignetes Multispender-Leberorgan wurde zuerst oberflächlich mit 5 L Phosphat-HEPES-Puffer mit doppelter Antibiotikakonzentration für 8 min. bei Raumtemperatur gespült und anschließend in zwei Schritten mit antibiotikahaltigen Puffern und in einem weiteren Schritt mit kollagenasehaltigem und antibiotikafreiem Puffer mit folgenden Volumina, pH 7,25 - 7,45, perfundiert:
1. 10 L EDTA-haltigem Phosphat-HEPES-Puffer; Perfusion 68 min; 20-40 °C;
2. 5 L Phosphat-HEPES Puffer; Perfusion 10 min; 25 - 40°C;
3. 3 L Kollagenase/Protease-haltigem und antibiotikafreiem Phosphat-HEPES Puffer; Perfusion 24 min; 25 - 40°C

Kollagenase/Protease-Konzentration: Kombination aus 2000 Wünsch-Einheiten Kollagenase I und 400 bis 2500 Einheiten Protease (Clostridiopeptidase I).

Die Perfusionszeit mit den Enzym- und Antibiotika-haltigen Puffern betrug insgesamt 102 min. Für die Perfusion wurde das natürliche Blutgefäßsystem der eingesetzten Leber genutzt.

Während der Perfusion wurde die Temperatur des perfundierten Organs und der Umgebung langsam von <10°C auf 37°C erhöht (Puffertemperatur zwischen Raumtemperatur und 40°C).

Die Zellen wurden nach ihrer Isolierung aus dem Gewebe mehrfach mit antibiotikafreiem Puffer gewaschen bis die Antibiotika-Konzentration unter 1/1000 der eingesetzten Antibiotika-Konzentration lag.

Eine Probe zur Sterilkontrolle wurde sowohl von dem Transportmedium, in dem das Organ geliefert wurde als auch von der desinfizierten Zellsuspension nach der Kryokonservierung entnommen.

### B. Ergebnisse

Im Transportmedium konnte Staphylococcus Aureus nachgewiesen werden. Die Zeit während der automatischen Inkubation, bis mikrobielles Wachstum festgestellt wurde, die so genannte "Time to positivity" betrug 3,85 h.

In der erfindungsgemäß desinfizierten Zellsuspension nach Kryokonservierung konnten auch nach 14-tägiger Bebrütung keine Keime nachgewiesen werden.

Nach einer ausreichenden Anzahl Waschschritte mit antibiotikafreiem Puffer kann mit den hergestellten Zellsuspensionen ein Steriltest durchgeführt werden.

## Patentansprüche

1. Verfahren zur Herstellung von desinfizierten Zellpräparaten aus Geweben von Säugern, umfassend die folgenden Verfahrensschritte:
a) Perfusion von Gewebe aus Säugern mit einer enzymfreien flüssigen in mindestens einem Perfusionspuffer vorliegenden, mindestens ein Antibiotikum aufweisenden antibiotischen Zusammensetzung,
b) Durchführung einer enzymatischen antibiotikafreien Behandlung des Gewebes zum Erhalt einer Einzelzellsuspension und
c) Erhalt von desinfizierten Zellpräparaten,
wobei die enzymatische Behandlung gemäß Schritt b) im Anschluss an Schritt a) erfolgt.

2. Verfahren nach Anspruch 1, wobei das Gewebe Lebergewebe ist.

3. Verfahren nach einem der Ansprüche 1 oder 2, wobei die enzymatische Behandlung eine Kollagenase- und/oder Proteinase-Behandlung ist.

4. Verfahren nach einem der Ansprüche 1 bis 3, wobei die Perfusionszeit in Verfahrensschritt a) 30 bis 120 min beträgt und die Temperatur während der Perfusion erhöht wird.

5. Verfahren nach einem der Ansprüche 1 bis 4, wobei die flüssige antibiotische Zusammensetzung in mindestens zwei Perfusionspuffern vorliegt, nämlich mindestens einem EGTA- oder EDTAhaltigen Phosphat-HEPES-Puffer und mindestens einem Phosphat-HEPES-Puffer.

6. Verfahren nach einem der vorhergehenden Ansprüche, wobei vor Verfahrensschritt a) die Oberfläche des Gewebes mit einer flüssigen antibiotischen Zusammensetzung gespült wird.

7. Verfahren nach einem der vorhergehenden Ansprüche, wobei im Anschluss an Verfahrensschritt b) mindestens ein Waschschritt mit einem Antibiotika-freien Waschpuffer durchgeführt wird.

## Claims

1. A process for the preparation of disinfected cell preparations from mammalian tissues, comprising the following process steps:
a) perfusing mammalian tissue with an enzyme-free liquid antibiotic composition that comprises at least one antibiotic and is contained in at least one perfusion buffer,
b) carrying out an enzymatic, antibiotic-free treatment of the tissue to obtain a single-cell suspension, and
c) obtaining disinfected cell preparations,
wherein the enzymatic treatment according to step b) takes place after step a).

2. The process according to claim 1, wherein the tissue is liver tissue.

3. The process according to either of claims 1 or 2, wherein the enzymatic treatment is a collagenase and/or proteinase treatment.

4. The process according to any one of claims 1 to 3, wherein the perfusion time in process step a) is 30 to 120 min and the temperature is increased during the perfusion.

5. The process according to any one of claims 1 to 4, wherein the liquid antibiotic composition is contained in at least two perfusion buffers, namely at least one EGTA- or EDTA-containing phosphate/HEPES buffer and at least one phosphate/HEPES buffer.

6. The process according to any one of the preceding claims, wherein the surface of the tissue is rinsed with a liquid antibiotic composition prior to process step a).

7. The process according to any one of the preceding claims, wherein at least one washing step using an antibiotic-free washing buffer is carried out after process step b).

## Revendications

1. Procédé de fabrication de préparations cellulaires désinfectées à partir de tissus de mammifères, comprenant les étapes de procédé suivantes :
a) perfusion de tissu de mammifères avec une composition antibiotique liquide exempte d'enzyme présente dans au moins un tampon de perfusion, présentant au moins un antibiotique,
b) réalisation d'un traitement enzymatique exempt d'antibiotique du tissu pour l'obtention d'une suspension unicellulaire et
c) obtention de préparations cellulaires désinfectées,
dans lequel le traitement enzymatique selon l'étape b) s'effectue à la suite de l'étape a).

2. Procédé selon la revendication 1, dans lequel le tissu est du tissu hépatique.

3. Procédé selon une des revendications 1 ou 2, dans lequel le traitement enzymatique est un traitement à base de collagénase et/ou protéinase.

4. Procédé selon une des revendications 1 à 3, dans lequel la durée de perfusion à l'étape de procédé a) se monte à 30 à 120 min et la température est augmentée au cours de la perfusion.

5. Procédé selon une des revendications 1 à 4, dans lequel la composition antibiotique liquide est présente dans au moins deux tampons de perfusion, à savoir au moins un tampon de phosphate-HEPES contenant EGTA ou EDTA et au moins un tampon de phosphate-HEPES.

6. Procédé selon une des revendications précédentes, dans lequel la surface du tissu est rincée avec une composition antibiotique liquide avant l'étape de procédé a).

7. Procédé selon une des revendications précédentes, dans lequel au moins une étape de lavage est réalisée avec un tampon de lavage exempt d'antibiotiques à la suite de l'étape de procédé b).
